(19) Europäisches Patentamt
European Patent Office
Office européen des brevets

(11) Publication number: 0 150 842 B1

# (12) EUROPEAN PATENT SPECIFICATION

(45) Date of publication of patent specification: 27.06.90

(51) Int. Cl.$^5$: C 07 D 303/22

(21) Application number: 85100863.1

(22) Date of filing: 28.01.85

## (54) Halogen-substituted Glycidyl ether compound and preparation process thereof.

(30) Priority: 27.01.84 JP 12001/84

(43) Date of publication of application: 07.08.85 Bulletin 85/32

(45) Publication of the grant of the patent: 27.06.90 Bulletin 90/26

(84) Designated Contracting States: CH DE FR GB IT LI NL SE

(56) References cited:
GB-A-1 093 840
US-A-3 833 522

The file contains technical information submitted after the application was filed and not included in this specification

(73) Proprietor: MITSUI TOATSU CHEMICALS, Inc.
2-5 Kasumigaseki 3-chome
Chiyoda-Ku Tokyo 100 (JP)

(72) Inventor: Itoh, Hiroshi
521, Kasamacho Totsuka-ku
Yokohama Kanagawa-ken (JP)
Inventor: Nitta, Atsuhiko
634-1-154, Nobacho Konan-ku
Yokohama Kanagawa-ken (JP)
Inventor: Tanaka, Tomio
8-14-2, Aoto Katsushika-ku
Tokyo (JP)
Inventor: Kamio, Hideo
728-5, Sagabetsusho Odawara
Kanagawa-ken (JP)
Inventor: Tsuboi, Kenji
4-5-45, Dai Kamakura
Kanagawa-ken (JP)

(74) Representative: Zumstein, Fritz jun., Dr. et al
Dr. F. Zumstein sen. Dr. E. Assmann Dr. R. Koenigsberger Dipl.-Ing. F. Klingseisen Dr. F. Zumstein jun. Bräuhausstrasse 4
D-8000 München 2 (DE)

EP 0 150 842 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

a) Field of the Invention

This invention relates to a novel halogen-substituted glycidyl ether compound having an ω-halogenopolymethylenoxy or ω-halogenoxylylenoxy group at one terminal of its molecule and a preparation process thereof.

### Summary of the Invention

An object of this invention is to provide a novel halogen-substituted glycidyl ether compound and a preparation process thereof.

The present invention provides the following halogen-substituted glycidyl ether compound and preparation process thereof:

A halogen-substituted glycidyl ether compound represented by the following general formula (1):

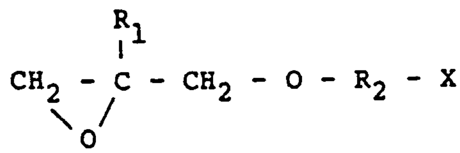 (1)

wherein $R_1$ means a hydrogen atom or methyl group,

$R_2$ denotes an alkylene group represented by the general formula $-(CH_2)_m-$, m standing for an integer of 4—12, or a xylylene group, and X is a halogen atom; and

A process for preparing the halogen-substituted glycidyl ether compound represented by the general formula (1), which process comprises reacting in an aprotic polar solvent and in the presence of a strong basic substance a hydroxyl-substituted compound, represented by the following general formula (2):

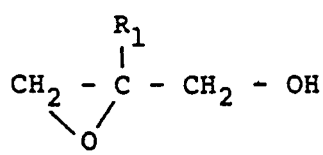 (2)

wherein $R_1$ has the same meaning as defined above in the general formula (1), with a dihalogen-substituted compound represented by the following general formula (3):

$$X—R_2—X \qquad (3)$$

wherein $R_2$ and X have the same meanings as defined above in the general formula (1).

### Detailed Description of the Invention

More specifically speaking, the compound of this invention, which is represented by the general formula (1), is obtained by substituting the hydroxyl group of glycidol or β-methylglycidol with an ω-halogenopolymethylenoxy or ω-halogenoxylylenoxy group. The xylylene group may be either one of its ortho, meta-, and para-forms. The halogen may for example be chlorine, bromine or iodine. Particularly preferred one is a compound of the general formula (1) in which $R_1$ means a hydrogen atom and m stands for an integer of 4—12.

The compounds of this invention are all novel compounds which have not been described in any literatures as far as the present inventors are aware of.

Certain representative examples of the compounds according to this invention will hereinafter be given. For the brevity and simplification of exemplification of compounds, compounds of the general formula (1) in which X is fixed to bromine and $R_2$ denotes polymethylene groups represented by the general formula: $-(CH_2)_m$[m: integer of 4—12] will first be mentioned by way of example, which will then be followed by exemplification of compounds of the general formula (1) in which X is fixed also to bromine but $R_2$ denotes a xylylene group.

First of all, as compounds in each of which $R_2$ is a polymethylene group, may for example be mentioned 1-bromo-4-glycidoxybutane, 1-bromo-5-glycidoxypentane, 1-bromo-6-glycidoxyhexane, 1-bromo, 8-glycidoxyoctane, 1-bromo-10-glycidoxydecane, 1-bromo-12-glycidoxydodecane, 1-bromo-4-(β-methyl)glycidoxybutane, 1-bromo-5-(β-methyl)glycidoxypentane, 1-bromo-6-(β-methyl)glycidoxyhexane, 1-bromo-8-(β-methyl)glycidoxyoctane, 1-bromo-10-(β-methyl)glycidoxydecane, 1-bromo-12-(β-methyl)-glycidoxydodecane, 1-iodo-4-glycidoxybutane, 1-iodo-6-glycidoxyhexane and 1-iodo-10-glycidoxydecane.

As compounds in each of which $R_2$ is a xylylene group, may for example be mentioned 1-bromomethyl-2-glycidoxymethylbenzene, 1-bromomethyl-3-glycidoxymethylbenzene, 1-bromomethyl-4-glycidoxy methylbenzene, 1-bromomethyl-2-(β-methyl)glycidoxymethylbenzene, 1-bromomethyl-3-(β-methyl)glycidoxymethylbenzene and 1-bromomethyl-4-(β-methyl)glycidoxymethylbenzene.

In the process of this invention which process is adapted to prepare the halogen-substituted glycidyl ether compound, the hydroxyl-substituted compound which is used as a starting material is either glycidol or β-methylglycidol, specifically speaking. On the other hand, the dihalogen-substituted compound is a straight-chain alkane in which both terminal C-atoms of its molecule have respectively been halogen-substituted or a xylene derivative in which each one of the hydrogen atoms of its two methyl groups has respectively been halogen-substituted. The xylene may be either one of its ortho-, meta- and para-forms. Each of the halogens may for example be chlorine, bromine or iodine.

As a specified method for effecting the reaction in the presence of the strong basic substance in the preparation process of this invention, the reaction may be initiated either by dissolving the strong basic substance in the liquid reaction mixture or by maintaining the strong basic substance in a state suspended in the liquid reaction mixture. Although both methods may be employed in the present invention, it is surprisingly preferred from the viewpoint of reaction efficiency such as suppression of side reactions to initiate the reaction with the strong basic substance maintained in a suspended state. More specifically, as a method for initiating the reaction while maintaining the strong basic substance in a suspended state, a suitable method may be employed. For example, it may be feasible to add at once the hydroxyl-substituted compound, dihalogen-substituted compound and strong basic substance to an aprotic polar solvent and then to stir the resultant mixture so as to suspend the strong basic substance for the initiation of the reaction; to suspend the strong basic substance in the aprotic polar solvent and then add the hydroxyl-substituted compound and dihalogen-substituted compound simultaneously to the resultant suspension for the initiation of the reaction; or to either dissolve or suspend the hydroxyl-substituted compound and dihalogen-substituted compound in the aprotic polar solvent and then to suspend the strong basic substance in the resultant solution or suspension for the initiation of the reaction.

The reaction solvent useful in the practice of the present invention may be any aprotic polar solvent. As exemplary aprotic polar solvents, may be mentioned acetonitrile, N,N-dimethylformamide, N,N-dimethylacetamide, dimethyl sulfoxide, sulfonane, tetraglime, dioxane and N-methylpyrrolidone.

In a reaction system in which the process of this invention is carried out, it is preferred to initiate the reaction with at least part of the strong basic substance being in a suspended state. The amount of water in such a state may generally be about 6 wt.% or below as the amount of water in the reaction system. If the amount of water should exceed the above-mentioned level, side reactions such as hydrolysis of the dihalogen-substituted compound tend to occur more readily, whereby to reduce the yield significantly. In order to carry out the reaction with good efficiency and hence to increase the yield of the intended reaction product, it is hence preferred to control the water content 5 wt.% or below in the reaction system.

No particular limitation is imposed on the amount of solvent to be used. It may however range from 5 wt.% to 95 wt.% or preferably, 10 wt.%—90 wt.%, both based on the total weight of the reaction mixture inclusive of the solvent.

Turning to the strong basic substance useful in the practice of this invention, any strong basic substance may be used so long as it is a solid form and when dissolved or suspended in water, provides an aqueous solution or suspension of at least pH 10 or preferably pH 11 or higher. As such basic substances, may for example be mentioned alkali metal hydroxides, alkali metal oxides, alkali metal carbonates, alkaline earth metal hydroxides, alkaline earth metal oxides, alkali metal hydrides, alkaline earth metal hydrides, alkali metal amides, and alkali metal alkoxides.

Illustrative of the above substances may include sodium hydroxide, potassium hydroxide, lithium hydroxide, rubidium hydroxide and cersium hydroxide as alkali metal hydroxides; sodium oxide, potassium oxide, lithium oxide, rubidium oxide and cesium oxide as alkali metal oxides; sodium carbonate, potassium carbonate, lithium carbonate, rubidium carbonate and cesium carbonate as alkali metal carbonates; beryllium hydroxide, magnesium hydroxide, calcium hydroxide, strontium hydroxide and barium hydroxide as alkaline earth hydroxides; beryllium oxide, magnesium oxide, calcium oxide, strontium oxide and barium oxide as alkaline earth metal oxides; sodium hydride, potassium hydride and lithium hydride as alkali metal hydrides; and beryllium hydride, magnesium hydride and calcium hydride as alkaline earth metal hydrides. The term "alkali metal amides" as used herein means alkali metal-substituted compounds of ammonia, including for example sodium amide, potassium amide and lithium amide. By the term "alkali metal alkoxides" as used herein is meant compounds obtained by substituting the protons of the hydroxyl groups of alcohols with alkali metals. Illustrative of such alkali metal alkoxides include sodium methoxide, sodium ethoxide, sodium t-butoxide, potassium methoxide, potassium ethoxide and potassium t-butoxide.

Among the above-described various basic substances, for example, alkali metal hydroxides, alkaline earth metal hydroxides, alkali metal oxides, alkaline earth metal oxides and alkali metal carbonates are suitable for the practice of the process of this invention.

Their strong basic substances are usually provided in their solid forms for the reaction. The reaction is initiated after a least part of such a strong basic substance has been rendered into a state suspended in the liquid reaction mixture.

In the practice of this invention, the relative proportions of the raw materials, i.e., the hydroxyl-substituted compound, dihalogen-substituted compounds and strong basic substance to be used may vary

depending on the reactivity between the dihalogen-substituted compound and the hydroxyl-substituted compound and the like and hence cannot be determined equally for different situations.

However, a side reaction takes place to substitute the two halogen atoms of the dihalogen-substituted compound and the yield is hence decreased if the relative ratio of the hydroxyl-substituted compound to the dihalogen-substituted compound approaches 1. On the other hand, existence of the dihalogen-substituted compound in large excess induces a reaction between the strong basic substance and dihalogen-substituted compound, whereby using up the dihalogen-substituted compound and thus resulting in a lowered yield. Roughly speaking, the dihalogen-substituted compound may be used in an amount within the range of 0.5—30 times or preferably 0.8—15 times in mole the hydroxyl-substituted compound. On the other hand, the strong basic substance may generally be used in an amount within the range of 0.3—15 times or preferably 0.5—10 times in mole the hydroxyl-substituted compound.

The reaction may be carried out using a usual reaction tank. When using a strong basic substance having low solubility, the reaction may be carried out in accordance with the flow reaction method, namely, by packing the strong basic substance in a tower and then causing a liquid mixture of the hydroxyl-substituted compound and dihalogen-substituted compound to pass through the tower while circulating the liquid mixture. For the maintenance and control of equipment, it is however more convenient to employ a reaction tank.

The reaction temperature is dependent on the reactivity between each hydroxyl-substituted compound and dihalogen-substituted compound to be used. However, the progress of the reaction will be retarded if the reaction temperature is unduly low. If the reaction temperature is excessively high, side reactions such as as hydrolysis of the dihalogen-substituted compound will occur and the yield of the intended reaction product will be reduced. Therefore, the reaction is usually carried out within the temperature range of −20—100°C, preferably −10—70°C or more preferably 0—50°C. Within these temperature ranges, it is not absolutely necessary to maintain the temperature at the same constant level during the reaction. The reaction may be efficiently caused to proceed by suitably controlling the reaction temperature while precisely observing the progress of the reaction.

Similar to the reaction temperature, the reaction time also varies in accordance with each hydroxyl-substituted compound and dihalogen-substituted compound to be employed. It may be within 30 hours at the longest, and usually within 20 hours. The progress of the reaction can be determined by observing changes in the nature of the reaction system and determining the concentrations of the raw materials and intended reaction product in the liquid reaction mixture by means of gas chromatography or high-speed liquid chromatography.

After the reaction, the resultant metal halide is filtered off for its separation. The filtrate is then distilled under reduced pressure by a method known *per se* in the art, thereby obtaining the intended reaction product with high purity. In case the metal halide is dissolved in the liquid reaction mixture or is otherwise allowed to remain in the liquid reaction mixture, after the solvent is distilled off, the metal halide is removed by a solvent extraction procedure using two solvents in combination, which solvents form two layers such as the combination of, for example, hexane-water, benzene-water or chloroform-water. Upon distillation of the organic layer under reduced pressure, the intended reaction product is obtained with high purity. If the intended reaction product has a high boiling point or is susceptible to decomposition under heat, the intended reaction product can be purified by such a method as, for example, solvent extraction or recrystallization.

If the reaction solvent has a high degree of miscibility with water like dimethyl sulfoxide and the intended reaction product is highly hydrophobic like a compound containing a long-chain polymethylene group, the intended reaction product may be isolated after the reaction, for example, by adding a solvent of aliphatic hydrocarbon such as hexane to the liquid reaction mixture to extract the intended product, adding water to the liquid reaction mixture to separate the intended product as an oil layer, or extracting the intended product with a solvent capable of forming two layers with water, such as benzene, toluene or chloroform.

According to the present invention, a wide variety of halogen-substituted glycidyl ether compounds can each be prepared through a one-step reaction and moreover, at a low cost. In addition, the preparation process of this invention permits preparation of a wide variety of halogen-substituted glycidyl ether compounds in the same reaction manner. Therefore, it is possible to prepare a broad variety of halogen-substituted glycidyl ether compounds by the same reaction. Hence, the preparation process of this invention has an advantage that it can be suitably used for the preparation of a variety of halogen-substituted glycidyl ether compounds in small volumes.

Since the halogen-substituted glycidyl ether compounds of this invention contain highly-reactive halogen atoms in their molecules, they may be reacted, similar to ordinary halogen-substituted compounds, with amide compounds, amines, hydroxyl-substituted compounds and the like. Since a glycidoxy or β-methylglycidoxy group is bonded as a functional group to one terminal of the molecule of each of the halogen-substituted glycidyl ether compounds, they can be used as extremely useful intermediates when introducing such functional groups into amide compounds, amines, hydroxyl-substituted compounds and the like.

The glycidyl ether compounds of this invention, in each of which a glycidoxy or β-methylglycidoxy group has been introduced, may be applied in a variety of fields, depending on their backbone structures.

Namely, as their specific application fields, they may be used as primary raw materials, auxiliary raw materials or crosslinking agents in adhesives, paints, paper-converting and conditioning agents, textile-processing agents, emulsions, urethane-curing agents, pigment dispersants, plastic additives, polymer flocculants, ion-exchange resins, absorber resins, chelate resins, optical lens, photosensitive resins, soft contact lens, hygroscopic resins, binders for magnetic materials, binder for composite materials, polymer catalysts, clinical reagents, bio-compatible materials, enzyme-immobilizing base materials, etc.

The invention will next be described further in the following Examples. It should however be borne in mind that the present invention will by no means be limited by or to the following Examples.

Example 1

Added to 150 ml of N,N-dimethylformamide were 11.4 g of glycidol and 81.0 g of 1,4-dibromobutane, followed by an addition with stirring of 7.8 g of flaky sodium hydroxide which had in advance been ground in a mortar. They were reacted at 25—30°C for 6 hours. After proceeded with the reaction for the predetermined period of time, insoluble matter was filtered off from the liquid reaction mixture and the solvent and unreacted raw materials were distilled off from the filtrate. The liquid residue was extracted with benzene-water, thereby obtaining the intended product in the benzene layer. The benzene was then distilled off from the benzene layer and the liquid residue was distilled under reduced pressure to obtain 24.5 g of 1-bromo-4-glycidoxybutane having a boiling point of 78—79°C/2.0 mmHg (yield: 78%). Its elementary analysis data(%) were as follows: Calculated: C, 40.20; H, 6.27; Br, 38.21. Found: C, 40.29; H, 6.07; Br 37.59.

Examples 2—5

Reactions were effected respectively with the combinations of raw materials, strong basic substances and solvents given in Table 1 and under the conditions also shown in Table 1. After the reactions, the resultant reaction mixtures were treated in exactly the same manner as in Example 1 to obtain the results given in Table 2.

Table 1

| Ex. | Hydroxyl-substituted compound (g) | Dihalogen-substituted compound (g) | Strong basic substance (g) | Solvent (ml) | Reaction temp. ($^{o}C$)/ reaction time(hrs.) |
|---|---|---|---|---|---|
| 2 | Glycidol (11.4) | 1,6-Dibromohexane (91.0) | Sodium hydroxide (7.8) | DMF (150) | 25-30/6 |
| 3 | Glycidol (11.4) | 1,8-Dibromooctane (101.2) | Sodium hydroxide (7.8) | DMF (150) | 25-30/6 |
| 4 | Glycidol (11.4) | 1,10-Dibromodecane (111.9) | Sodium hydroxide (7.8) | DMF (150) | 25-30/6 |
| 5 | Glycidol (11.4) | 1,12-Dibromododecane (122.0) | Sodium hydroxide (7.8) | DMF (150) | 25-30/6 |

Table 2

| Ex. | Reaction product | Boiling point (°C/mmHg) | Elementary analysis data(%), (calculated) C | H | Br | Yield,g (%) |
|---|---|---|---|---|---|---|
| 2 | 1-Bromo-6-glycidoxyhexane | 104-105/1.0 | 45.30 (45.57) | 6.82 (7.23) | 34.27 (33.69) | 26.0 (73) |
| 3 | 1-Bromo-8-glycidoxyoctane | 95-97/0.05 | 48.80 (49.80) | 8.39 (7.99) | 31.26 (30.12) | 26.3 (66) |
| 4 | 1-Bromo-10-glycidoxydecane | 111-112/0.1 | 52.26 (53.23) | 9.09 (8.60) | 27.02 (27.24) | 26.8 (61) |
| 5 | 1-Bromo-12-glycidoxydodecane | 127-128/0.07 | 55.26 (56.06) | 9.27 (9.11) | 25.26 (24.86) | 25.5 (53) |

Example 6

Added to 50 ml of N,N-dimethylformamide were 1.9 g of glycidol and 16.5 g of p-xylylenedibromide, followed by an addition with stirring of 1.3 g of flaky sodium hydroxide which had in advance been ground in a mortar. They were reacted at 25—30°C for 6 hours. After proceeded with the reaction for the predetermined period of time, insoluble matter was filtered off from the liquid reaction mixture and the solvent and unreacted raw materials were distilled off from the filtrate.

The liquid residue was extracted with benzene-water, thereby obtaining the intended product in the benzene layer. The benzene was then distilled off from the benzene layer to obtain 3.3 g of 1-bromomethyl-4-glycidoxymethylbenzene (yield: 52%). It was then purified by column chromatography, using silica gel as an absorbant and benzene as a developer solvent. Measurement of melting point and elementary analysis were then performed on the thus-purified product. The following results were obtained. Melting point: 57—59°C. Elementary analysis data(%): Calculated: C, 51.37; H, 5.10; Br 31.07. Found: C, 50,93; H, 5.53; Br, 30.88.

Example 7

Added to 50 ml of N,N-dimethylformamide were 1.5 g of glycidol and 13.7 of n-xylylenedibromide, followed by an addition with stirring of 2.2 g of flaky sodium hydroxide which has in advance been ground in a mortar. They were reacted at 15—20°C for 7 hours. After proceeded with the reaction for the predetermined period of time, insoluble matter was filtered off from the liquid reaction mixture and the solvent and unreacted raw materials were distilled off from the filtrate.

The liquid residue was extracted with benzene-water, thereby obtaining the intended product in the benzene layer. The benzene was then distilled off from the benzene layer to obtain 2.2 g of 1-bromomethyl-3-glycidoxymethylbenzene (yield: 41%). It was then purified by column chromatography, using silica gel as an adsorbent and benzene as a developer solvent. Measurement of refractive index at 25°C and elementary analysis were then performed on the thus-purified product. The following results were obtained. Refractive Index: 1.5585. Elementary analysis data(%): Calculated: C, 51.37; H, 5.10; Br, 31.07. Found: C, 51.87; H, 5.23; Br, 31.66.

Example 8

Added to 150 ml of N,N-dimethylformamide were 4.44 g of glycidol and 50.70 g of 1,6-diiodohexane, followed by an addition with stirring of 3.36 g of flaky sodium hydroxide which had in advance been ground in a mortar. They were reacted at 15—20°C for 8 hours. After proceeded with the reaction for the predetermined period of time, insoluble matter was filtered off from the liquid reaction mixture. The filtrate was washed with h-hexane to extract and remove unreacted 1,6-diiodohexane, and the solvent was then distilled off.

The residue was extracted with benzene-water, thereby obtaining the intended product in the benzene layer. The benzene was then distilled off from the benzene layer to obtain 12.7 g of 1-iodo-6-glycidoxyhexane (yield: 74%). It was then purified by column chromatography, using silica gel as an adsorbent and a benzene-methanol mixed solvent as a developer solvent. Measurement of refractive index at 25°C and elementary analysis were then performed on the thus-purified product. The following results were obtained. Refractive Index (25°C): 1.5322. Elementary analysis data(%): Calculated: C, 38.04; H, 6.03; I, 44.66. Found: C, 37.97; H, 6.07; I, 44.49.

Example 9

Added to 150 ml of N,N-dimethylformamide were 3.70 g of glycidol and 49.26 g of 1,10-diiodododecane, followed by addition with stirring of 2.80 g of flaky sodium hydroxide which had in advance been ground in a mortar. They were reacted at 15—20°C for 8 hours. After proceeded with the reaction for the predetermined period of time, the reaction mixture was treated in exactly the same manner as in Example 8 to obtain 13.0 g of 1-iodo-10-glycidoxydecane (yield: 76%). It was then purified in exactly the same manner as in Example 8. Measurement of refractive index at 25°C and elementary analysis were then performed on the thus-purified product. The following results were obtained. Refractive Index (25°C): 1.5100. Elementary analysis data(%): Calculated: C, 45.89; H, 7.41; I, 37.30. Found: C, 46.02; H, 7.36; I, 37.19.

Example 10

Added to 150 ml of N,N-dimethylformamide were 4.44 g of glycidol and 46.49 g of 1,4-diiodobutane, followed by an addition with stirring of 3.36 g of flaky sodium hydroxide which had in advance been ground in a mortar. They were reacted at 15—20°C for 7 hours. After proceeded with the reaction for the predetermined period of time, the reaction mixture was treated in exactly the same manner as in Example 8 to obtain 11.2 g of 1-iodo-4-glycidoxybutane (yield: 73%). It was then purified in exactly the same manner as in Example 8. Measurement of refractive index at 25°C and elementary analysis were then performed on the thus-purified product. The following results were obtained. Refractive Index (25°C): 1.5744. Elementary analysis data(%): Calculated: C, 32.83; H, 5.12; I, 49.56. Found: C, 32.79; H, 5.09; I, 49.62.

**Claims**

1. A halogen-substituted glycidyl ether compound represented by the following general formula (1):

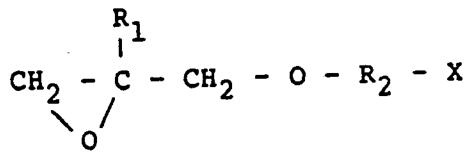

(1)

wherein $R_1$ means a hydrogen atom or methyl group, $R_2$ denotes an alkylene group represented by the general formula $-(CH_2)_m-$, m standing for an integer of 4—12, or a xylylene group, and X is a halogen atom.

2. A process for preparing halogen-substituted glycidal ether compound represented by the general formula (1):

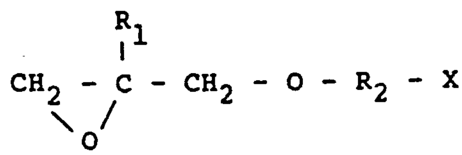

(1)

wherein $R_1$ means a hydrogen atom or methyl group, $R_2$ denotes an alkylene group represented by the general formula $-(CH_2)_m-$, m standing for an integer of 4—12, or a xylylene group, and X is a halogen atom, which process comprises reacting in an aprotic polar solvent and in the presence of a strong basic substance a hydroxyl-substituted compound, represented by the following general formula (2):

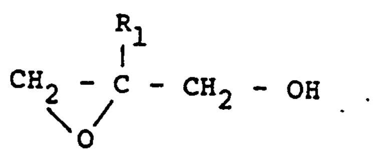

(2)

wherein $R_1$ has the same meaning as defined above, with a dihalogen-substituted compound represented by the following general formula (3):

$$X—R_2—X \qquad (3)$$

wherein $R_2$ and X have the same meanings as defined above.

**Patentansprüche**

1. Halogensubstituierte Glycidyletherverbindung der folgenden allgemeinen Formel (1)

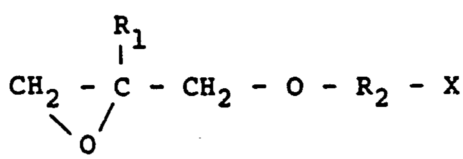

(1)

worin $R_1$ für ein Wasserstoffatom oder eine Methylgruppe steht, $R_2$ eine Alkylengruppe der allgemeinen Formel

$$-(CH_2)_m-$$

bedeutet, worin m für eine ganze Zahl von 4 bis 12 steht, oder eine Xylylengruppe bezeichnet, und X ein Halogenatom ist.

2. Verfahren zur Herstellung einer halogensubstituierten Glycidyletherverbindung der folgenden allgemeinen Formel (1)

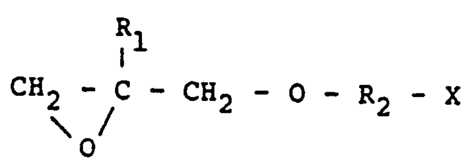

(1)

worin $R_1$ für ein Wasserstoffatom oder eine Methylgruppe steht, $R_2$ eine Alkylengruppe der allgemeinen Formel

$$-(CH_2)_m-$$

bedeutet, worin m für eine ganze Zahl von 4 bis 12 steht, oder eine Xylylengruppe bezeichnet, und X ein Halogenatom ist, das die Umsetzung einer hydroxyl-substituierten Verbindung der folgenden allgemeinen Formel (2)

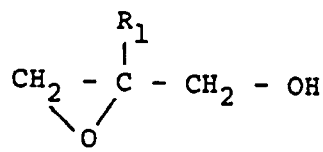

(2)

worin $R_1$ die vorstehend angegebene Bedeutung besitzt, mit einer dihalogen-substituierten Verbindung der folgenden allgemeinen Formel (3)

$$X—R_2—X \qquad (3)$$

worin $R_2$ und X wie vorstehend definiert sind, in einem einem aprotischen Lösungsmittel und in Gegenwart einer stark basischen Substanz umfaßt.

**Revendications**

1. Composé d'éther glycidylique halogéno-substitué représenté par la formule générale (1) suivante:

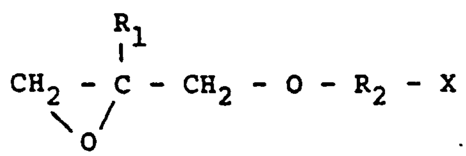

(1)

dans laquelle $R_1$ représente un atome d'hydrogène ou un groupe méthyle, $R_2$ désigne un groupe alkylène représenté par la formule générale $-(CH_2)_m-$, m étant un nombre entier de 4 à 12, ou un groupe xylylène, et X est un atome d'halogène.

2. Procédé de préparation d'un composé d'éther glycidylique halogéno-substitué représenté par la formule générale (1) suivante:

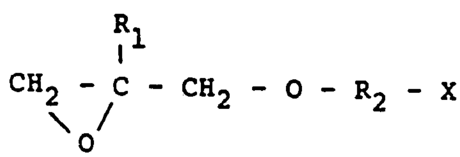

(1)

dans laquelle $R_1$ représente un atome d'hydrogène ou un groupe méthyle, $R_2$ désigne un groupe alkylène représenté par la formule générale $-(CH_2)_m-$, m étant un nombre entier de 4 à 12, ou un groupe xylylène, et X est un atome d'halogène, lequel procédé comprend la réaction dans un solvant polaire aprotique et en présence d'une substance basique forte d'un composé hydroxyl-substitué représenté par la formule générale (2) suivante:

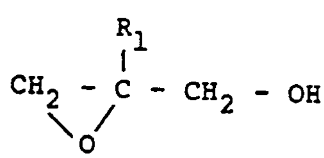

(2)

dans laquelle $R_1$ a la même signification que celle définie ci-dessus, avec un composé dihalogéno-substitué représenté par la formule générale (3) suivante:

$$X—R_2—X \qquad (3)$$

dans laquelle $R_2$ et X ont les mêmes significations que celles définies ci-dessus.